# EUROPEAN PATENT APPLICATION

(11) **EP 3 875 083 A1**
(43) Date of publication of application: **08.09.2021**
(21) Application number: 20160817.1
(22) Date of filing: 03.03.2020
(51) Int. Cl.: A61K 31/194, A61K 31/19, A61K 31/185, A61K 33/04, A61P 35/00, A61K 9/00, A61K 31/191, A61P 31/12, A61K 9/06

(54) **COMPOSITION FOR USE IN A TREATMENT OF CERVICAL CELL ABNORMALITIES COMPRISING SELENITE COMPOUND AND ACID**

(71) Applicant: Selo Medical GmbH, 5585 Unternberg (AT)
(72) Inventor: FUCHS, Norbert, 5580 Unternberg (AT)
(74) Representative: SONN Patentanwälte OG

(57) **Abstract**

The present invention provides a pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids and mixtures thereof, for use in reducing progression of cervical cell abnormalities in a female patient, wherein the patient is pl6-positive and Ki-67-positive at least in a region of the cervix uteri. The composition is applied intravaginally.

## Description

The present invention relates to a composition for use in a treatment of cervical cell abnormalities.

Cervical smear screening (also known as Pap test or Pap smear) is a widely used practice for detecting potentially precancerous and cancerous processes in the cervix uteri of female patients. Typically, cervical cell abnormalities are classified according to the Bethesda system (see Nayar R, Wilbur D. The Bethesda System for Reporting Cervical Cytology, Definitions, Criteria, and Explanatory Notes. Springer; 2015.). Such abnormalities include atypical squamous cells of undetermined significance (ASC-US), atypical squamous cells - cannot exclude HSIL (ASC-H), atypical glandular cells (AGC), low-grade squamous intraepithelial lesion (LSIL) and high-grade squamous intraepithelial lesion (HSIL), among others. It is known that these abnormalities can improve but also deteriorate with time. In this connection, remission is defined as a complete recovery from abnormal cytological smear findings, regression is defined as an improvement of cytological smear findings (e.g. from HSIL to LSIL or ASC-US, or from LSIL to ASC-US), persistence is defined as no change of cytological smear findings and progression is defined as a worsening of cytological smear findings (e.g. from ASC-US to ASC-H or LSIL, or from ASC-H to LSIL or from LSIL to HSIL).

Depending on the severity of the cervical cell abnormalities, and often after a period of "watchful waiting" to assess whether the abnormalities will remit or regress spontaneously (or, by contrast, will persist or even progress), surgical interventions are recommended according to common treatment guidelines. Typical procedures include cone-shaped excision, cryotherapy, loop electrosurgical excision procedure (LEEP) or large loop excision of the cervical transformation zone (LLETZ).

As any surgical intervention confers a certain health risk, there is a need for non-surgical interventions to reduce the progression rates of cervical cell abnormalities, in order to decrease the need for surgery or at least the invasiveness of the surgery should such an intervention still be necessary.

It is hence an object of the present invention to provide a non-surgical treatment which reduces progression rates of cervical cell abnormalities.

The present invention relates to a pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids (e.g. malic acid, citric acid, tartaric acid, oxalic acid and fumaric acid, in particular citric acid) and mixtures thereof. The composition is for use in reducing progression of cervical cell abnormalities (typically as determined by cervical smear screening) in a female patient. The composition is applied intravaginally to the patient. In a particularly preferred embodiment, the patient is p16-positive and Ki-67-positive (i.e. p16/Ki-67-double-positive) at least in a region of the cervix uteri, and/or at least a portion of the cervical cell abnormalities is p16-positive and Ki-67-positive (i.e. p16/Ki-67-double-positive).

In the course of the present invention, it was surprisingly found that the pharmaceutical composition is not only effective in increasing remission and regression of the cervical cell abnormalities, but remarkably also in reducing progression of cervical cell abnormalities. This was confirmed by a controlled clinical trial (see example 2).

While the pharmaceutical composition was previously disclosed for use against other therapeutic indications, these disclosures do not anticipate or suggest the present invention:
US 2003/0180387 A1 relates to a method for increasing the antioxidative potential of selenium-containing aqueous solutions. It is disclosed that a preparation comprising a pharmaceutically administrable or food-compatible form of selenium, namely selenite, and a pharmaceutically acceptable or food-compatible acid, selected from citric acid, acetic acid, malic acid, carbonic acid, various fruit acids and mixtures thereof, can be used to prevent or treat herpes simplex infections, among many other indications. However, the document neither discloses intravaginal application nor that the target organ is the cervix uteri nor the inventive therapeutic indication.

US 2005/0048134 A1 concerns the use of a selenite-containing compound to be topically or buccally administered. Treatment or prophylaxis of infections with papilloma viruses, particularly in the genital region, is disclosed as one among many indications. However, the document neither discloses intravaginal application nor that the target organ is the cervix uteri nor the inventive therapeutic indication.

US 2013/0323328 A1 relates to a pharmaceutical preparation containing selenite or selenite-containing compounds for treating cervical dysplasia or carcinomas. However, the document is silent at least with respect to the inventive therapeutic indication.

In a particularly preferred embodiment of the present invention, the cervical cell abnormalities to be treated are selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US, all according to the Bethesda system (see also explanation and book citation above).

In embodiments, the patient has cervical cell abnormalities selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US; in particular as determined by cervical smear screening (especially of said region of the cervix uteri).

According to a preference, said progression of cervical cell abnormalities comprises (or: is further defined as) at least one of change from ASC-US to ASC-H, change from ASC-US to AGC, change from ASC-US to LSIL, change from ASC-US to HSIL, change from ASC-H to AGC, change from ASC-H to LSIL, change from ASC-H to HSIL, change from AGC to LSIL, change from AGC to HSIL and change from LSIL to HSIL, preferably at least one of change from ASC-US to ASC-H, change from ASC-US to AGC, change from ASC-US to LSIL, change from ASC-US to HSIL, change from ASC-H to AGC, change from ASC-H to LSIL and change from ASC-H to HSIL, more preferably at least one of change from ASC-US to ASC-H, change from ASC-US to AGC, change from ASC-US to LSIL and change from ASC-US to HSIL, in particular at least one of change from ASC-US to ASC-H and change from ASC-US to LSIL. Preferably, such a change is determined after at least 30 days, preferably at least 60 days, more preferably at least 90 days, even more preferably at least 120 days, yet even more preferably at least 150 days or even at least 180 days have passed after initial screening which may e.g. performed when starting the inventive treatment.

In the context of the present invention, reducing progression is preferably defined as not comprising increasing remission and increasing regression.

p16 (also known as p16^{INK4a} or cyclin-dependent kinase inhibitor 2A) is a protein that is encoded by the *CDKN2A* gene in humans. Overexpression of p16 is a biomarker of increased cervical cancer risk. Ki-67 (also known as KI-67 or MKI67) is a protein that is encoded by the *MKI67* gene in humans. Ki-67 is a biomarker for cell proliferation. Both of these biomarkers may be tested for instance with the CINtec® PLUS test (Roche, Switzerland) which is a commercially available, approved cytological assay (see e.g. Ravarino, A., et al. (2012). CINtec PLUS immunocytochemistry as a tool for the cytologic diagnosis of glandular lesions of the cervix uteri. American Journal of Clinical Pathology, 138(5), 652-656).

In the course of the present invention, it was found that the inventive therapy is especially effective in reducing progression of cervical cell abnormalities when they are associated with biomarkers p16 and Ki-67. Accordingly, in a preferred embodiment of the present invention, the patient is p16-positive, preferably p16-positive and Ki-67-positive, at least in a region of the cervix uteri (and/or at least a portion of the cervical cell abnormalities is p16-positive and/or Ki-67-positive). Preferably, the pharmaceutical composition is applied until the patient has become p16-negative, preferably p16-negative and Ki-67-negative, in said region (and/or until at least a portion of the cervical cell abnormalities has become p16-negative and/or Ki-67-negative).

The skilled person knows how to assess whether a region of the cervix uteri (or at least a portion of the cell abnormalities) is p16-positive, e.g. by performing a biopsy in the region and using a p16 assay (such as an immunocytochemistry assay) known in the art. Preferably, a biopsy sample obtained from the region of the cervix uteri is defined as p16-positive if at least one cell, preferably at least two adjoining cells, more preferably at least three adjoining cells, even more preferably at least five adjoining cells, in particular at least ten adjoining cells over-express p16 compared to healthy control tissue, such as healthy adjoining cells in the biopsy sample, preferably wherein each of said cells are epithelial cells. Conversely, such a biopsy sample is preferably defined as p16-negative if less than ten adjoining cells, preferably less than five adjoining cells, more preferably less than three adjoining cells, even more preferably less than two cells, in particular no cell over-expresses p16 compared to healthy control tissue, such as healthy adjoining cells in the biopsy sample, preferably wherein each of said cells are epithelial cells.

Further, the skilled person also knows how to assess whether a region of the cervix uteri (or at least a portion of the cell abnormalities) is Ki-67-positive, e.g. by performing a biopsy in the region and using a Ki-67 assay (such as an immunocytochemistry assay) known in the art. Preferably, a biopsy sample obtained from the region of the cervix uteri is defined as Ki-67-positive if at least one cell, preferably at least two adjoining cells, more preferably at least three adjoining cells, even more preferably at least five adjoining cells, in particular at least ten adjoining cells over-express Ki-67 compared to healthy control tissue, such as healthy adjoining cells in the biopsy sample, preferably wherein each of said cells are epithelial cells. Conversely, such a biopsy sample is preferably defined as Ki-67-negative if less than ten adjoining cells, preferably less than five adjoining cells, more preferably less than three adjoining cells, even more preferably less than two cells, in particular no cell over-expresses Ki-67 compared to healthy control tissue, such as healthy adjoining cells in the biopsy sample, preferably wherein each of said cells are epithelial cells.

In a particular preference, "p16-positive and Ki-67-positive" is defined as a positive result in the established CINtec® PLUS test (Roche, Switzerland), especially as disclosed in CINtec® PLUS Interpretation Guide (Roche, 2016). In this Interpretation Guide, a positive result is defined as the presence of at least one dual-stained cervical epithelial cell (the cytoplasm stains brown (p16) and the nucleus stains red (Ki-67)).

In the course of the present invention, it turned out that the inventive composition is effective already at surprisingly low concentrations of selenium. Therefore, in a further preferred embodiment, the total selenium content of the pharmaceutical composition is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition. It is evident that "total selenium content" does not imply that selenium has to be present as elemental selenium in the composition. By way of example, 0.83 mg sodium selenite as the only selenium-containing compound per 5 ml of composition corresponds to a total selenium content ("selenium-equivalent content") of 0.25 mg per 5 ml.

It was also found that the selenium dose per application can be low and still be effective. According to a further preferred embodiment, the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application. It is evident that "total selenium dose" does not imply that selenium has to be present as elemental selenium in the dose. By way of example, 0.83 mg sodium selenite as the only selenium-containing compound of a dosage unit applied corresponds to a total selenium dose ("selenium-equivalent dose") of 0.25 mg per application.

For increased effectiveness, it is preferred that the pharmaceutical composition is applied at least once per day, preferably for at least 30 days, more preferably for at least 60 days, even more preferably for at least 90 days.

It has turned out that application of the composition only once per day is suitable for achieving effectiveness. As this further reduces the risk of side effects compared to several applications per day, a further preferred embodiment relates to the inventive pharmaceutical composition for use wherein the composition is applied once per day, preferably for at least 30 days, more preferably for at least 60 days, even more preferably for at least 90 days.

The application of the pharmaceutical composition may be discontinued during menstruation of the patient.

The pharmaceutical composition for use according to the present invention may additionally contain further suitable ingredients and/or pharmaceutically acceptable excipients.

Preferably, the pharmaceutical composition for use according to the present invention contains selenite in the form of sodium selenite (which is mostly present as a pentahydrate compound which starts to release crystal water at 40°C).

In a further preferred embodiment of the present invention, the composition contains one or more acids in a total amount between 1 mg and 10 g acid, more preferably between 10 mg and 5 g acid, in particular between 100 mg and 1 g acid, per 100 g of the composition (in particular if the acid is added in its solid form). Alternatively, the acid may also be added in its liquid form (e. g. with water, i.e. as an aqueous solution). Water and aqueous solutions, respectively, optionally containing further ingredients, may be added to the composition according to the present invention in an amount between 0 and (about) 99.9 g, preferably between 50 and 99 g, in particular between 80 and 98g, per 100 g of the composition.

According to a further preferred embodiment, the composition is present in the form of a gel, a suspension, an emulsion, a suppository such as gelatine capsules or gelatine-free capsules, a spray or a powder.

When present in the form of a gel, the pharmaceutical composition for use according to the present invention preferably contains a gelling agent. Both inorganic and organic aqueous gelling agents may be used as a gelling agent. Particularly suitable gelling agents are cellulose derivatives, in particular carboxymethylcellulose, methylcellulose, hydroxypropyl cellulose and, in particular, hydroxyethyl cellulose. Preferably, the gelling agents, in particular hydroxyethyl cellulose, are used at a total concentration of between 0.1 g and 30 g, more preferably between 0.5 g and 5 g, in particular between 1 g and 3 g, per 100 g of the composition.

A further particularly preferred embodiment of the composition for use according to the present invention, especially when the composition is present in the form of a gel, contains silicon dioxide, in particular highly dispersed silicon dioxide, e. g. according to WO 2001/85852 A1, as a technological suspension medium and/or as an adsorbent. Preferably, an amount between 100 mg and 50 g, more preferably between 500 mg and 10 g, in particular between 1 g and 5 g, SiO2 per 100 g of the composition is used.

The composition for use according to the present invention preferably has a pH-value of less than 7.0, more preferably less than 5.0, in particular between 4.0 and 2.5.

Advantageously, the composition may contain further excipients and/or further active ingredients, in particular buffer substances, colouring agents, stabilizers, preservatives, carrier substances or combinations thereof. Preferred examples of preservatives are potassium sorbate and sodium benzoate.

Moreover, the composition may additionally comprise further active agents such as antibiotics, antiviral agents, antimycotics, pain inhibitors, anti-inflammatory agents or combinations thereof.

The present invention also relates to a method for reducing progression of cervical cell abnormalities in a female patient (preferably wherein the patient is p16-positive and Ki-67-positive at least in a region of the cervix uteri), comprising
- obtaining a pharmaceutical composition as defined herein; and
- administering an effective amount of the composition to the patient, wherein the composition is applied intravaginally.

Typically, the patient is in need of the inventive treatment.

According to a particular preference, the human papillomavirus (HPV) infection status of said region of the cervix uteri, preferably of the entire cervix uteri, of the patient is unknown or is not determined. In addition, or alternatively thereto said region of the cervix uteri, preferably the entire cervix uteri, is preferably not infected with any HPV selected from HPV16, HPV18, HPV31, HPV33 and HPV58 (preferably with neither of the five).

HPV infections may be specifically detected e.g. by polymerase chain reaction (PCR) or transcription-mediated amplification (TMA) assays of biopsy samples such as cervical smears, or other methods known in the art, since the genome sequences of HPV16, HPV18, HPV31, HPV33 and HPV58 are known. Genome sequences of these HPV types are for instance published in National Center for Biotechnology Information (NCBI) GenBank under the following accession numbers: K02718.1 (HPV16), X05015.1 (HPV18), J04353.1 (HPV31), M12732.1 (HPV33) and D90400.1 (HPV58).

Herein, the patient to be subject to inventive treatment is preferably older than 20 years, preferably older than 30 years, even more preferably older than 40 years, yet even more preferably older than 50 years. The patient is human.

The inventive treatment has been found to be not as efficient in immunosuppressed patients. Therefore, the patient is preferably not immunosuppressed. In addition, or alternatively thereto, the patient preferably does not have cancer and/or chronic viral disease (or chronic viral disease other than HPV infection).

Herein, the term "over-express" or similar in regard to a gene (product) A typically can mean that the expression level of A (as e.g. measured by Western blot or immunohistochemistry or immunocytochemistry) e.g. in a biopsy sample is higher than the expression level of an appropriate control (such as healthy tissue of the same type), by a factor of at least 1.2 (i.e. an increase of at least 20%), preferably at least 1.4, more preferably at least 1.6, even more preferably at least 1.8, especially at least 2.0.

The present invention is further illustrated by the following figures and examples, without being restricted thereto.

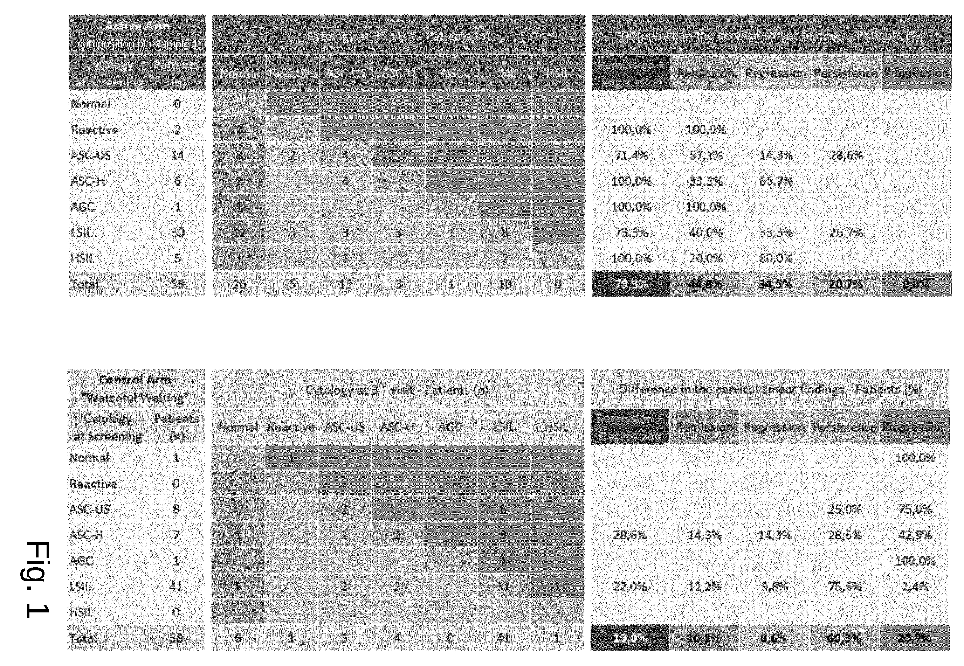

**Fig. 1****: Difference in the cervical smear findings** - **Screening vs. 3^{rd} visit.** An interim analysis showed that compared to the non-treated control arm the progression rate was strongly reduced in the active arm treated with the inventive therapy (0.0% in the active arm vs. 20.7% in the control arm). "at Screening": initial screening of the patient, "at 3^{rd} Visit": after 3x28 days of once-daily treatment.

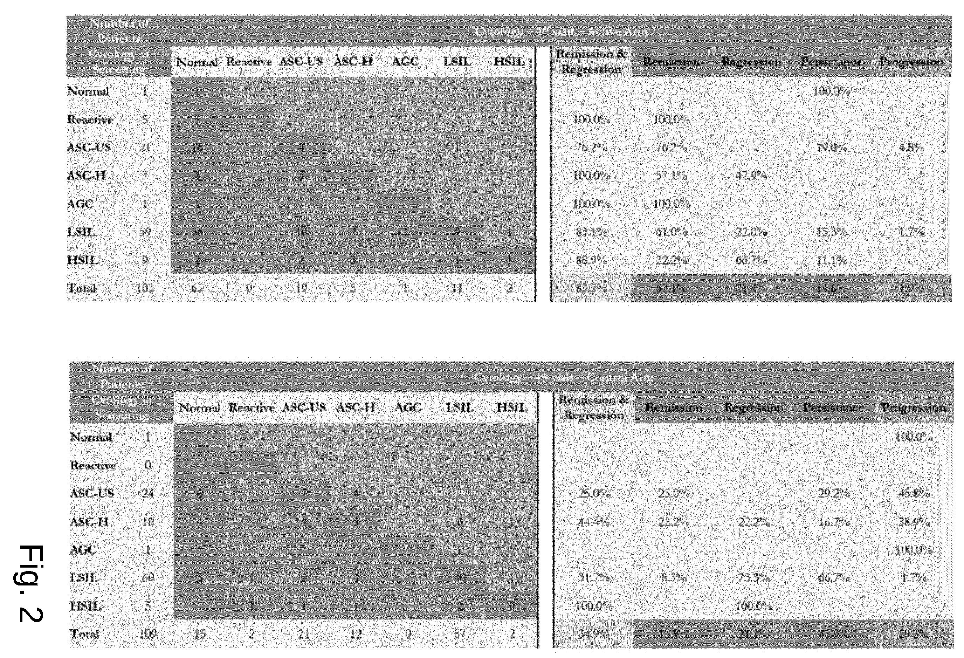

**Fig. 2****: Difference in the cervical smear findings** - **Screening vs. 4^{th} visit.** Compared to the non-treated control arm the progression rate remained strongly reduced in the active arm treated with the inventive therapy even months after the treatment had ended (1.9% in the active arm vs. 19.3% in the control arm) "at Screening": initial screening of the patient, "at 4^{th} Visit": six months after initial screening.

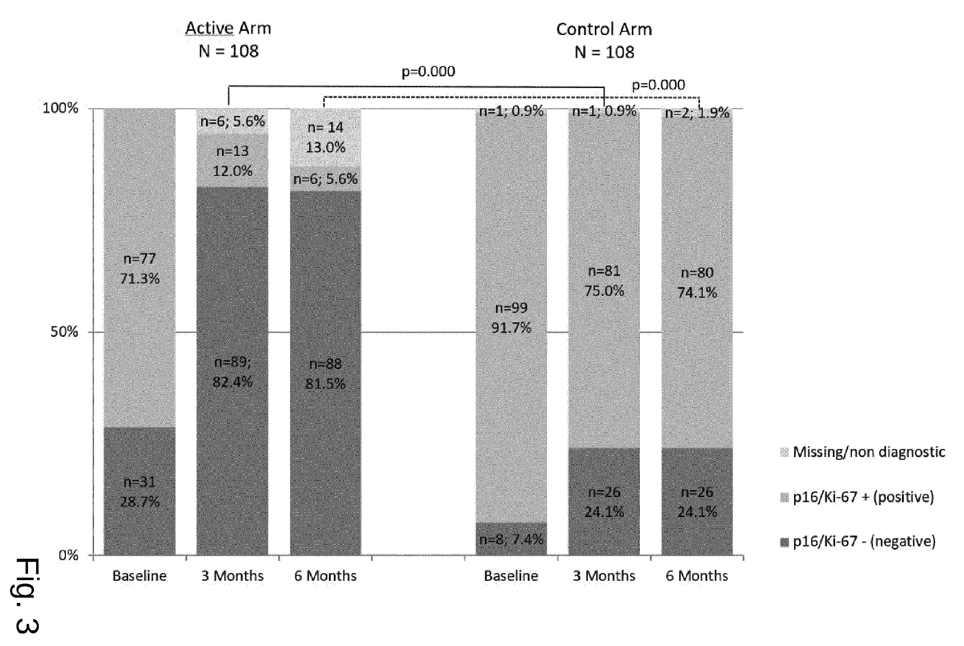

**Fig. 3****: Improvement of p16/Ki-67 status.** A much larger remission rate from p116/Ki-67 double-stain positive was observed in the active arm treated with the inventive therapy, even months after the treatment had ended. "p16/Ki-67 -": negative as determined with the established CINtec® PLUS test (Roche, Switzerland). "p16/Ki-67 +": positive as determined with the established CINtec® PLUS test (Roche, Switzerland).

### Example 1 - Pharmaceutical composition for use in reducing progression of cervical cell abnormalities

The composition is an aqueous vaginal gel having the following ingredients (per 5ml of gel):

| | |
|---|---|
| highly dispersed silicon dioxide | 10.00 mg |
| citric acid | 24.80 mg |
| sodium selenite | 0.83 mg |
| (corresponds to 0.25 mg selenium equivalent) | |
| sodium benzoate | 2.50 mg |
| potassium sorbate | 5.00 mg |
| hydroxyethyl cellulose | 100.00 mg |
| water | (up to 5 ml) |

### Example 2 - Controlled clinical trial

The present trial was a randomized, prospective, open label with control group multicentre study of 3 x 28 days once a day treatment with the composition of example 1 (female patients, age 25-60 years, no chronic viral disease, cancer and immunosuppressive treatment). Daily dose was 5 ml of the composition, applied intravaginally.

The subjects were recruited in 3 centres. Cervical smears were collected according the standard procedure known in the art, preserved in a specific liquid milieu called SurePath® (Becton & Dickinson) and evaluated in accordance to the Bethesda classification. Samples were further analysed with the CINtec® PLUS cytology kit (Roche, Switzerland).

An interim analysis was conducted after about 50% of patients (N = 111; 50% evaluable per arm) had completed about 3 months in the trial (see Fig. 1). Final evaluations with larger patient sets were conducted at the end of the trial, 3 months after the treatment had ended (see Figs. 2 and 3).

### Results

Evaluated data demonstrate a clearly improved outcome for patients in the active arm for all measured parameters. Only a few slight adverse events were reported.

Compared to the non-treated control arm the progression rate was strongly reduced in the active arm treated with the inventive therapy (see Fig. 1, 0.0% in the active arm vs. 20.7% in the control arm), and remained strongly reduced even months after the treatment had ended (see Fig. 2, 1.9% in the active arm vs. 19.3% in the control arm).

Further, compared to the non-treated control arm, a much larger remission rate from p16/Ki-67 double-stain positive was observed in the active arm treated with the inventive therapy (see Fig. 3).

These results demonstrate the exceptional suitability of the composition for the therapeutic indications mentioned herein.

## Claims

1. A pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids and mixtures thereof, for use in reducing progression of cervical cell abnormalities in a female patient, wherein the patient is p16-positive and Ki-67-positive at least in a region of the cervix uteri, wherein the composition is applied intravaginally.

2. The pharmaceutical composition for use according to claim 1, wherein said cervical cell abnormalities are selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US.

3. The pharmaceutical composition for use according to claim 1 or 2, wherein the composition is applied until the patient has become p16-negative, preferably p16-negative and Ki-67-negative, in said region.

4. The pharmaceutical composition for use according to any one of claims 1 to 3, wherein the total selenium content is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition; and/or wherein the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application.

5. The pharmaceutical composition for use according to any one of claims 1 to 4, wherein the composition is applied at least once per day, preferably for at least 30 days, more preferably for at least 60 days, even more preferably for at least 90 days; optionally wherein the application is discontinued during menstruation of the patient.

6. The pharmaceutical composition for use according to any one of claims 1 to 5, wherein the composition is applied once per day, optionally wherein the application is discontinued during menstruation of the patient.

7. The pharmaceutical composition for use according to any one of claims 1 to 6, wherein the application is discontinued during menstruation of the patient; and/or wherein the application is discontinued for at least 30 days, more preferably at least 60 days, even more preferably at least 90 days after treatment for at least 30 days, more preferably for at least 60 days, even more preferably for at least 90 days, optionally wherein the application is discontinued during menstruation of the patient.

8. The pharmaceutical composition for use according to any one of claims 1 to 7, wherein said progression of cervical cell abnormalities comprises at least one of change from ASC-US to ASC-H, change from ASC-US to AGC, change from ASC-US to LSIL, change from ASC-US to HSIL, change from ASC-H to AGC, change from ASC-H to LSIL, change from ASC-H to HSIL, change from AGC to LSIL, change from AGC to HSIL and change from LSIL to HSIL, preferably at least one of change from ASC-US to ASC-H, change from ASC-US to AGC, change from ASC-US to LSIL, change from ASC-US to HSIL, change from ASC-H to AGC, change from ASC-H to LSIL and change from ASC-H to HSIL, more preferably at least one of change from ASC-US to ASC-H, change from ASC-US to AGC, change from ASC-US to LSIL and change from ASC-US to HSIL, in particular at least one of change from ASC-US to ASC-H and change from ASC-US to LSIL.

9. The pharmaceutical composition for use according to any one of claims 1 to 8, wherein the composition is present in the form of a gel, a suspension, an emulsion, a suppository such as gelatine capsules or gelatine-free capsules, a spray or a powder.

10. The pharmaceutical composition for use according to any one of claims 1 to 9, wherein the composition further contains silicon dioxide, preferably highly disperse silicon dioxide.

11. The pharmaceutical composition for use according to any one of claims 1 to 10, wherein the composition has a pH of less than 7.0, preferably less than 5.0, in particular between 4.0 and 2.5.

12. The pharmaceutical composition for use according to any one of claims 1 to 11, wherein the pharmaceutically acceptable acid is citric acid.

13. The pharmaceutical composition for use according to any one of claims 1 to 12, wherein the selenite-containing compound is sodium selenite.

14. The pharmaceutical composition for use according to any one of claims 1 to 13, wherein human papillomavirus (HPV) infection status of said region of the cervix uteri, preferably of the entire cervix uteri, of the patient is unknown or is not determined; or wherein said region of the cervix uteri, preferably the entire cervix uteri, is not infected with any HPV selected from HPV16, HPV18, HPV31, HPV33 and HPV58, preferably not with any HPV.

15. The pharmaceutical composition for use according to any one of claims 1 to 14, wherein the patient does not have cancer, and/or chronic viral disease or chronic viral disease other than HPV infection, and/or wherein the patient is not immunosuppressed.
